# DEMANDE DE BREVET EUROPEEN

(11) **EP 1 870 077 A2**
(43) Date de publication de la demande: **26.12.2007**
(21) Numéro de dépôt: 07109959.2
(22) Date de dépôt: 11.06.2007
(51) Int. Cl.: A61K 8/11, A61K 8/894, A61Q 17/04

(54) **Utilisation cosmétique de particules concaves ou annulaires de matériau silicone comme agent permettant d'augmenter le facteur de protection solaire ; composition photoprotectrice aqueuse les contenant**

(30) Priorité: 22.06.2006 FR 0652593
(71) Demandeur: L'Oréal, 75008 Paris (FR)
(72) Inventeur: Simonnet, Jean-Thierry, 94240, CACHAN (FR); L'Alloret, Florence, 75014, PARIS (FR)
(74) Mandataire: Miszputen, Laurent

(57) **Abrégé**

La présente invention concerne l'utilisation de particules concaves ou annulaires de matériau siliconé, notamment sous forme de portions de sphères creuses dans une composition comprenant dans un milieu physiologiquement acceptable au moins une phase aqueuse et au moins un filtre UV organique et/ou un filtre UV minéral, dans le but d'augmenter le facteur de protection solaire (FPS).

La présente invention a aussi pour objet l'utilisation de particules concaves ou annulaires de matériau siliconé, notamment sous forme de portions de sphères creuses associées à au moins un agent mouillant dans une composition comprenant dans un milieu physiologiquement acceptable au moins une phase aqueuse et au moins un filtre UV organique et/ou un filtre UV minéral, dans le but de réduire la brillance après application.

L' invention concerne aussi une composition comprenant dans un milieu physiologiquement acceptable :
a) au moins une phase aqueuse
b) des particules concaves ou annulaires de matériau siliconé, notamment sous forme de portions de sphères creuses ;
c) au moins un agent mouillant ;
d) au moins un filtre UV organique et/ou un filtre UV minéral.

## Description

La présente invention a pour objet l'utilisation de particules concaves ou annulaires de matériau siliconé, notamment sous forme de portions de sphères creuses dans une composition comprenant dans un milieu physiologiquement acceptable au moins une phase aqueuse et au moins un filtre UV organique et/ou un filtre UV minéral, dans le but d'augmenter le facteur de protection solaire (FPS).

La présente demande concerne plus particulièrement une composition comprenant dans un milieu physiologiquement acceptable:
a) au moins une phase aqueuse
b) des particules concaves ou annulaires de matériau siliconé, notamment sous forme de portions de sphères creuses ;
c) au moins un agent mouillant ;
c) au moins un filtre UV organique et/ou un filtre UV minéral.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et/ou UVB dans le cadre de la photoprotection quotidienne et solaire.

Cependant, l'un des objectifs majeurs de ces applications est, pour des raisons d'agrément cosmétique, de tolérance mais aussi pour des raisons de formulation de limiter autant que faire ce peut le taux de filtres solaires dans la composition.

Dans le brevet US 6039935, on a déjà proposé des formulations solaires sous forme d'émulsion huile/eau comprenant des filtres UV organiques et une silicone élastomèrique réticulée non émulsionnante et silicone volatile pour augmenter le niveau de protection et minimiser la quantité de filtre. Il s'agit de microgels huileux de silicone élastomérique réticulé qui ont pour inconvénients de modifier la viscosité de la composition.

On cherchera donc des matériaux, qui introduits dans la composition permettent non seulement d'améliorer significativement l'efficacité des filtres solaires mais sans les inconvénients énoncés ci-dessus notamment sans augmentation de la viscosité et permettant d'obtenir un touchers sec non gras.

On connaît dans l'art antérieur notamment dans les demandes de brevet JP-A-2000-191789, JP-A-2003-128788, EP1579849, EP1579841, FR2867677 et FR2877839 des particules concaves de matériau d'organopolysiloxane réticulé qui ont la forme de portions de sphères creuses, obtenues par condensation de silanols résultant de l'hydrolyse de composés organosiliconés. Dans ces demandes, il est indiqué de les utiliser notamment dans des produits cosmétiques pour le visage ou dans des produits de maquillage, en particulier dans des poudres compactes de fond de teint. des sticks de rouge à lèvres. Dans les demandes de brevet EP1642619 et EP1642620, ces mêmes particules ont été proposées dans des compositions capillaires de fixation et/ou de maintien de la coiffure. On connaît également des particules siliconées de forme annulaire dans la demande de brevet US2006/0089778 pouvant être utilisées comme poudre dans des produits pour le soin du visage, des produits de maquillage, des produits pour le corps, des produits capillaires ou des déodorants.

La Demanderesse a découvert de manière surprenante et inattendue, que cet objectif pouvait être atteint en utilisant des particules concaves de matériau siliconé dans une composition aqueuse comprenant au moins un filtre UV organique et/ou un filtre UV minéral

Par ailleurs, la demanderesse a constaté que ces particules concaves de matériau siliconé en association avec un agent mouillant dans une composition aqueuse comprenant au moins un filtre UV organique et/ou un filtre UV minéral, permettaient également d'augmenter significativement l'efficacité photoprotectrice qui se traduit notamment par le facteur de protection solaire (FPS).

La présente invention a donc pour objet l'utilisation de particules concaves ou annulaires de matériau siliconé, notamment sous forme de portions de sphères creuses dans une composition aqueuse comprenant dans un milieu physiologiquement acceptable au moins une phase aqueuse et au moins un filtre UV organique et/ou un filtre UV minéral, dans le but d'augmenter le facteur de protection solaire (FPS).

La présente invention a aussi pour objet l'utilisation de particules concaves ou annulaires de matériau siliconé, notamment sous forme de portions de sphères creuses en association avec un agent mouillant dans une composition aqueuse comprenant dans un milieu physiologiquement acceptable au moins un filtre UV organique et/ou un filtre UV minéral, dans le but d'augmenter le facteur de protection solaire (FPS).

La présente invention a également pour objet une composition aqueuse comprenant dans un milieu physiologiquement acceptable :
a) des particules concaves ou annulaires de matériau siliconé, notamment sous forme de portions de sphères creuses ;
b) au moins un agent mouillant ;
c) au moins un filtre UV organique et/ou un filtre UV minéral.

On entend par «milieu physiologiquement acceptable», un milieu non toxique et susceptible d'être appliqué sur la peau, les lèvres, les cheveux, les cils, les sourcils les ongles. La composition de l'invention peut constituer notamment une composition cosmétique ou dermatologique.

Par « agent mouillant», on entend tout composé qui, introduit en solution aqueuse à 0,05% (en poids) permet de réduire la tension superficielle de l'eau à une valeur inférieure à 35 mN/m, et de préférence inférieure à 30 mN/m.

Le facteur de protection solaire (FPS) s'exprime mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV.

On entend par « composition comprenant au moins une phase aqueuse » par une formulation du type émulsion huile-dans-eau (H/E) ou eau-dans-huile (E/H) ou multiple (triple : E/H/E ou H/E/H). La proportion massique de la phase aqueuse varie de préférence de 25 à 95%, de préférence de 30 à 90% et de façon encore plus préférentielle de 40 à 80%. On choisira plus particulièrement les émulsions du type huile-dans-eau ou du type eau-dans-huile-dans-eau.

### Particules concaves ou annulaires de matériau siliconé

Les particules concaves ou annulaires présentes dans la composition selon l'invention sont des particules siliconées, en particulier des particules de portions de sphères creuses constituées d'un matériau siliconé.

Lesdites particules ont de préférence un diamètre moyen inférieur ou égal à 10 µm, notamment allant de 0,1 µm à 8 µm, préférentiellement de 0,2 à 7 µm, plus préférentiellement allant de 0,5 à 6 µm, et de préférence encore allant de 0,5 à 4 µm.

Par diamètre moyen, on entend la plus grande dimension de la particule.

De façon avantageuse, ces particules ont une densité supérieure à 1.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau siliconé est un polysiloxane réticulé de structure tridimensionnelle , il comprend de préférence, voire est constitué de, des motifs de formule (l) SiO₂ et de formule (II) R¹SiO_{1,5}
dans lesquels R¹ désigne un groupe organique ayant un atome de carbone directement relié à l'atome de silicium.

Le groupe organique R¹ peut être un groupe organique réactif ; R¹ peut être plus particulièrement un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano, et de préférence, un groupe époxy, un groupe (méth)acryloxy, un groupe alkényle, un groupe mercaptoalkyle, aminoalkyle. Ces groupes comprennent généralement de 2 à 6 atomes de carbone, notamment de 2 à 4 atomes de carbone.

Le groupe organique R¹ peut être aussi un groupe organique non réactif ; R¹ peut être alors plus particulièrement un groupe alkyle en C₁-C₄, notamment un groupe méthyle, éthyle, propyle, butyle, ou un groupe phényle, et de préférence un groupe méthyle.

Comme groupe époxy, on peut citer un groupe 2-glycidoxyéthyle, un groupe 3-glycidoxypropyle, un groupe 2-(3,4-époxycyclohexyl)propyle

Comme groupe (méth)acryloxy, on peut citer un groupe 3-méthacryloxypropyl, un groupe 3-acryloxypropyle.

Comme groupe alkényle, on peut citer les groupes vinyle, allyle, isopropényle.

Comme groupe mercaptoalkyle, on peut citer les groupes mercaptopropyle, mercaptoéthyle.

Comme groupe aminoalkyle, on peut citer un groupe 3-(2-aminoéthyl)aminopropyle, un groupe 3-aminopropyl, un groupe N,N-diméthylaminopropyle.

Comme groupe halogénoalkyle, on peut citer un groupe 3-chloropropyle, un groupe trifluoropropyle.

Comme groupe glycéroxy, on peut citer un groupe 3-glycéroxypropyl, un groupe 2-glycéroxyéthyle.

Comme groupe uréido, on peut citer un groupe 2-uréidoéthyle.

Comme groupe cyano, on peut citer les groupes cyanopropyle, cyanoéthyle.

De préférence, dans le motif de formule (II), R¹ désigne un groupe méthyle.

Avantageusement, le matériau siliconé comprend les motifs (I) et (II) selon un rapport molaire motif (I) / motif (II) allant de 30/70 à 50/50, de préférence allant de 35/65 à 45/55.

Les particules de matériau siliconé peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant des groupements alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

Pour les groupements X et Y des composés (III) et (IV):

Comme groupe alcoxy en C₁-C₄, on peut citer les groupes méthoxy, éthoxy ;

Comme groupe alkoxyéthoxy renfermant un groupe alcoxy en C₁-C₄, on peut citer les groupes méthoxyéthoxy, butoxyéthoxy ;

Comme groupe alcoxy en C₂-C₄, on peut citer les groupes acétoxy, propioxy ;

Comme groupe N,N-dilakylamino renfermant un groupement alkyle en C₁-C₄, on peut citer les groupes diméthylamino, diéthylamino ;

Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemples de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemples de composés de formule (IV) comportant un groupe R organique réactif, on peut citer :
- les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
- les silanes ayant un groupe (méth)acryloxy comme le 3-méthacryloxypropyl triméthoxysilane, le 3-acryloxypropyl triméthoxysilane ;
- les silanes ayant un groupe alkényle comme le vinyl triméthoxysilane, l'allyl triméthoxysilane, l'isopropényl triméthoxysilane ;
- les silanes ayant un groupe mercapto comme le mercaptopropyl triméthoxysilane, le mercaptoéthyl triméthoxysilane ;
- les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
- les silanes ayant un groupe halogénoalkyl comme le 3-chloropropyl triméthoxysilane, le trifluoropropyl triméthoxysilane ;
- les silanes ayant un groupe glycéroxy comme le 3-glycéroxypropyl triméthoxysilane, le di(3-glycéroxypropyl)diméthoxysilane ;
- les silanes ayant un groupe uréido comme le 3-uréïdopropyl triméthoxysilane, le 3-uréïdopropyl méthyldiméthoxysilane, le 3-uréïdopropyl diméthyl-méthoxysilane ;
- les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques tels que l'hydroxyde de sodium, l'hydroxyde de potassium, le carbonate de sodium, l'hydrogénocarbonate de sodium, l'ammoniaque ou des amines telles que la triméthylamine, la triéthylamine, l'hydroxyde de tétraméthylammonium, ou des catalyseurs acides tels que les acides organiques comme l'acide citrique, l'acide acétique, l'acide méthanesulfonique, l'acide p-toluène sulfonique, l'acide dodécylbenzène-sulfonique, l'acide dodécylsulfonique, ou les acides minéraux tels que l'acide chlorhydrique, l'acide sulfurique, l'acide phosphorique.

Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-A-2003-128788.

Des portions de sphères creuses en forme de fer à cheval sont décrites dans la demande JP-A-2000-191789.

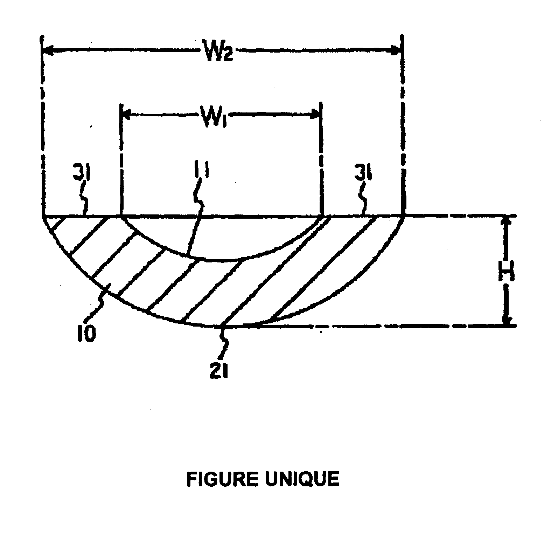

La figure 1 annexée illustre une particule concave sous forme de portions de sphères en forme de bol en coupe transversale. La largeur W2 correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules concaves sous forme de portions de sphères utilisables selon l'invention, on peut citer par exemple :
- les particules constituées de l'organosilicone réticulé TAK-110 (polymère réticulé méthylsilanol/silicate) de la société TAKEMOTO OIL & FAT, en forme de bol, de largeur 2,5 µm, de hauteur 1,2 µm et d'épaisseur 150 nm (particules vendues sous la dénomination NLK-506 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 0,8 µm, de hauteur 0,4 µm et d'épaisseur 130 nm (particules vendues sous la dénomination NLK-515 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 7 µm, de hauteur 3,5 µm et d'épaisseur 200 nm (particules vendues sous la dénomination NLK-510 par la société Takemoto Oil & Fat).
   Ces particules ont pour nom INCI : Methylesilanol/silicate crosspolymer.
   Avantageusement, les particules concaves siliconées ont un diamètre moyen inférieur ou égal à 5 µm, notamment allant de 0,1 µm à 5 µm, préférentiellement allant de 0,2 à 5 µm, plus préférentiellement allant de 0,5 à 4 µm, et de préférence encore allant de 0,5 à 3 µm.
   Ces particules permettent, outre la réduction voire l'élimination du toucher collant, l'optimisation des propriétés de glissant, d'étalement, et de confort de la composition selon l'invention.
   Les particules siliconées de forme annulaire sont de préférence choisies parmi celles décrites et synthétisées dans la demande US-A-2006/0089478. Elles présentent un diamètre moyen extérieur de 0,05 à 15 µm et un diamètre moyen intérieur de 001 à 10 µm; la différence entre le diamètre moyen extérieur et le diamètre moyen intérieur étant de 0,04 à 5 µm.
   Elles présentent un réseau polysiloxane comprenant des unités siloxane de formule (1), (2) (3) (4) (5) et (6)

   SiO_{4/2} (1)

   Si(OH)_{3/2} (2)

   R₁(SiO_{3/2} (3)

   R₂SiO_{3/2} (4)

   R₃SiO_{3/2} (5)

   R₄SiO_{3/2} (6)
dans lesquelles
- R₁ et R₃ désignent des groupes hydrocarbonés non réactifs, notamment des groupes alkyle, cycloalkyle, aryle, alkylaryle ou aralkyle, de préférence les groupes alkyles en C₁-C₃ notamment méthyle, éthyle, propyle, et préférentiellement un groupe méthyle
   et R₂ et R₄ désignent chacun un groupe hydrocarboné choisi parmi les groupes acryloxy, methacryloxy, vinyle ou mercapto ;
   le rapport molaire unités siloxane de formule (1) / unités siloxane de formule (2), (3), (4), (5) et (6) étant de 20/80 à 50/50 ;
   le rapport molaire unités siloxane de formule (2), (3) et (4) / unités siloxane de formule (5) et (6) étant de 50/50 à 75/25;
   le rapport molaire unités siloxane de formule (3) et (5) / unités siloxane de formule (4) et (6) étant de 20/80 à 60/40.
   Comme groupe acryloxy, on peut citer un groupe 2-méthacryloxyéthyle, un groupe 3-acryloxypropyl.
   Comme groupe (méth)acryloxy, on peut citer un groupe 3-méthacryloxypropyl, un groupe 3-acryloxypropyle.
   Comme groupe mercaptoalkyle, on peut citer un groupe mercaptopropyl, mercaptoéthyl.
   Comme groupe vinyle, on peut citer les groupes allyle, isopropenyle, 2-methylallyle.
   Les particules concaves ou annulaires siliconées peuvent être présentes dans la composition selon l'invention en une teneur allant de 0,1 à 15 % en poids, de préférence allant de 0,5 à 10 % en poids, et préférentiellement allant de 0,5 à 7,5 % en poids par rapport au poids total de la composition.

### Filtres UV

Les compositions filtrantes conformes à l'invention comportent des filtres UV organiques et/ou inorganiques actifs dans l'UVA et/ou l'UVB hydrophiles et/ou lipophiles et/ou bien insolubles dans les solvants cosmétiques couramment utilisés.

Les filtres UV organiques hydrophiles, lipophiles ou insolubles sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate , les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine) et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

-1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Les filtres UV organiques préférentiels sont choisis parmi
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine
la 2,4,6-tris(biphenyl-4-yl-1,3,5-triazine)
la 2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres inorganiques sont choisis parmi des pigments (taille moyenne des particules primaires: généralement entre 5 nm et 100 nm, de préférence entre 10 nm et 50 nm) d'oxydes métalliques enrobés ou non comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et «Mirasun TiW 60» de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 T, MT 100 TX, MT 100 Z, MT-01 de la société TAYCA, les produits "Solaveil CT-10 W" et "Solaveil CT 100" de la société UNIQEMA et le produit "Eusolex T-AVO" de la société MERCK,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.
   D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.
   Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".
   Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".
Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les filtres UV sont généralement présents dans les compositions selon l'invention dans des proportions allant de 0,01 à 20% en poids par rapport au poids total de la composition, et de préférence allant de 0,1 à 10% en poids par rapport au poids total de la composition.

Selon une forme particulièrement préféré de l'invention, les compositions comprennent en plus au moins un agent mouillant.

Les agents mouillants conformes à l'invention sont choisis de préférence parmi les silicones hydrosolubles comportant au moins un groupement polyoxyalkyléné monovalent terminal ou pendant et qui, introduits à 0,05% en poids dans une solution aqueuse, sont susceptibles de réduire la tension superficielle de l'eau à une valeur inférieure à 35 mN/m, et de préférence inférieure à 30 mN/m.

Les agents mouillants conformes à l'invention sont choisis plus préférentiellement parmi les silicones hydrosolubles comportant au moins un groupement polyoxyalkyléné de formule générale (a) suivante

R²₃SiO(R²₂SiO)ₚ(R²PESiO)_{q}SIR²₃ (a)

dans laquelle
- les radicaux R² identiques ou différents, désignent un radical hydrocarboné monovalent choisis parmi les radicaux alkyles, aryles et aralkyles ayant au plus 10 atomes de carbone ; certains des radicaux R² peuvent également contenir en plus un groupe ethylcyclohexylenemonoxyde de formule 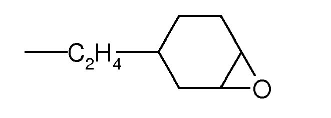 et sont en faible proportion dans la chaîne polysiloxane ;
- p varie de 0 à 150, de préférence de 0 à 100 et plus préférentiellement de 0 à 30
- q varie de 1 à 12, de préférence de 1 à 10 et plus préférentiellement de 1 à 8.
- le groupe polyéther PE a la formule (b) suivante

   -CₓH₂ₓ(OC₂H₄)_{y}(OC₃H₆)_{z}OR³ (b)
dans laquelle :
x varie de 1 à 8 et de préférence varie de 2 à 4 et plus préférentiellement est égal à3;
y est supérieur à 0
z est supérieur ou égal à 0 ; les valeurs de y et z sont tels que le poids moléculaire total de la portion polyoxyalkylénée du groupe polyéther E varie de 200 à 10.000 et plus préférentiellement de 350 à 4000 ;
R³ désigne hydrogène, un groupe alkyle en C₁-C₈ ou un groupe acyle en C₂-C₈ .

Il est à noter que lorsque z est différent de 0, les unités polyoxyéthylène et polyoxypropylène peuvent être distribuées de manière aléatoire le long de la chaîne polyéther PE ou répartis en blocs ou bien à la fois répartis en blocs et de manière aléatoire.

De préférence, les radicaux R² sont choisis parmi es alkyles inférieurs en C₁-C₆ comme méthyle, éthyle, butyle, hexyle; phényle et benzyle. Et plus particulièrement, les radicaux R² sont choisis parmi les alkyles inférieurs en C₁-C₄ et encore plus particulièrement désignent méthyle.

De préférence, les radicaux R³ sont choisis parmi les alkyles inférieurs en C₁-C₄ et encore plus particulièrement désignent méthyle.

Le nombre d'unités d'oxyéthylene du groupe E doit être suffisant pour produire un point de turbidité dans l'eau entre 25 et 90°C et plus préférentiellement de 40 à 70 °C.

Les silicones hydrosolubles de formule (a) peuvent être obtenues selon le procédé décrit dans le brevet US4,847398.

Parmi les silicones hydrosolubles de formule (a), on utilisera de préférence celle de formule (a') suivante :

MeSiO(MeSiO)ₚ(MePESiO)_{q}SlMe₃ (a')

où Me désigne méthyle ; PE désigne :

-(CH₂)₃O(OC₂H₄)_{y}(OC₃H₆)_{z}OR³ (b')

où x, z et z ont les mêmes valeurs indiquées ci-dessus et R³ désigne hydrogène ou un groupe alkyle en C₁-C₄. et plus particulièrement methyle

Comme autre famille de silicones hydrosolubles utilisables selon l'invention, on peut citer les silicones ramifiées de formule (c) suivante :

(MeSiO)_{q-2}[SiOMe2)_{p/q} OPE]_{q} (c)

où p, q ont les mêmes valeurs indiquées ci-dessus dans la formule (a) ; Me signifie méthyle ;PE désigne le groupe de formule (d) suivante :

-(OC₂H₄)_{y}(OC₃H₆)_{z}R³ (d)

où y et z ont les mêmes valeurs indiquées ci-dessus dans la formule (b) et R³ désigne un groupe alkyle en C₁-C₄. et plus particulièrement méthyle.

De telles silicones sont par exemple vendues par la société OSI sous les dénominations commerciales Silwet L-720® , Silwet L-7002®, Silwet L-7600® , Silwet L-7604® , Silwet L-7605® , Silwet L-7607®, Silwet 1614, Silwet L-7657® , Silwet L-7200®, Silwet L7230, Silsoft 305, Silsoft 820, Silsoft 880 ou encore par la société Goldschmidt sous les dénominations commerciales Tego wet 260, Tegowet 500, Tegowet 505 et Tegowet 510®.

Le tableau suivant rassemble des valeurs de tensions superficielles à 25°C de solutions aqueuses comprenant 0,05% (en poids) de différents agents mouillants.

| **Agent mouillant** | **Tension superficielle à 0,05% dans l'eau (mN/m), 25°C** |
|---|---|
| Tegowet 500 | 33 |
| Tegowet 510 | 29 |
| Silsoft 880 | 26 |
| Silsoft 305 | 21 |

Selon l'invention, le ou les agents mouillants sont présents à des concentrations allant de 0,01 % à 10% en poids, de préférence de 0,05% à 5% en poids et plus particulièrement de 0,1 à 3% en poids par rapport au poids total de la composition finale.

Les compositions selon l'invention peuvent être préparées selon les techniques de préparation des émulsions bien connues de l'homme de l'art. Elles se présentent généralement sous forme d'émulsion, simple ou complexe (H/E, E/H, H/E/H ou E/H/E).

Les compositions utilisables selon l'invention peuvent être plus ou moins fluides et avoir l'aspect d'une crème blanche ou colorée, d'une pommade, d'un lait, d'une lotion, d'un sérum, ou d'une pâte. Elles peuvent aussi se présenter sous forme solide, et par exemple sous forme de stick. Elles peuvent éventuellement être conditionnées en aérosol et se présenter sous forme de mousse ou de spray.

On choisira plus particulièrement les émulsions du type huile-dans-eau ou du type eau-dans-huile-dans-eau.

Les compositions conformes à l'invention comportent généralement au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les esters comme les benzoates d'alcools gras en C12-C15 (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

La phase huileuse peut aussi comporter un ou plusieurs corps gras choisi par exemple parmi les alcools gras (alcool cétylique, l'alcool stéarylique, alcool cétéarylique), les acides gras (acide stéarique) ou les cires (paraffine, cires de polyéthylène, carnauba, cire d'abeilles). La phase huileuse peut contenir des gélifiants lipophiles, des tensio-actifs, ou encore des particules organiques ou minérales. La phase huileuse peut représenter de préférence de 1 à 70% d'huile en poids par rapport au poids total de la composition.

Les compositions selon l'invention sous forme d'émulsion comprennent généralement des tensioactifs émulsionnants qui sont choisis de manière appropriée suivant le type d'émulsion choisie. Quand l'émulsion est triple, elle comporte généralement un émulsionnant dans l'émulsion primaire et un émulsionnant dans la phase externe dans laquelle est introduite l'émulsion primaire.

Dans le cas de l'émulsion simple huile dans eau, la phase huileuse dispersée peut représenter de 1 à 70% en poids, de préférence de 5 à 60% en poids et mieux de 10 à 50% en poids par rapport au poids total de l'émulsion simple.

Dans le cas de l'émulsion triple eau dans huile dans eau, l'émulsion primaire E/H peut représenter par exemple de 5 à 70 % en poids, de préférence de 10 à 60 % en poids et mieux de 15 à 50 % en poids par rapport au poids total de l'émulsion triple. La phase aqueuse interne de l'émulsion primaire E/H représente de préférence de 5 à 90 %, mieux de 30 à 90 % et encore mieux de 40 à 80 % du poids total de l'émulsion primaire.

Les tensioactifs émulsionnants sont généralement présents à une concentration minimale de 0,5% en poids par rapport au poids total de la composition.

Ils sont de préférence utilisés à des proportions allant de 0,5 à 30 % en poids, de préférence de 0,5 à 20 % en poids et mieux de 0,5 à 15 % en poids par rapport au poids total de la composition.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI ;

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés); les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Selon un mode particulier de réalisation de l'invention, les émulsions selon l'invention peuvent comporter seulement 1% en poids ou moins, et même être exemptes de tensioactifs émulsionnants, tout en étant stables au stockage. Dans ce cas, elles peuvent être stabilisées par différentes techniques telles que l'utilisation d'agents épaississants hydrophiles ou lipophiles comme ceux du brevet EP864320, de polymères amphiphiles telles que ceux cités dans le brevet EP1093796, dans la demande de brevet WO02/44231, de particules solides (émulsions type Pickering) telles que les émulsions citées dans les demandes de brevet WO98/42300, WO98/42301, EP987001,EP987002, EP987003, EP987004 EP987005 EP987006 EP-987007 EP-987008, WO2000/07548, WO2000/07549, EP992233.

Dans les émulsions triples E/H/E selon l'invention, l'émulsion primaire E/H comporte de façon avantageuse au moins un émulsionnant de HLB inférieure à 10 (HLB = Hydrophilic Lipophilic Balance = Balance hydrophile lipophile). Les émulsionnants peuvent être choisis par exemple dans le groupe comprenant les alcools gras alcoxylés et notamment éthoxylés, les esters gras alcoxylés et notamment éthoxylés, les esters ou éthers glycérolés (comme par exemple l'isostéarate de polyglycérol-4), les sels d'acides gras tels que le Stéarate d'Aluminium, les tensioactifs dérivés de sucre tels que l'isostéarate de méthylglucose, les tensio-actifs polymériques dérivés de polyoléfines et les émulsionnants siliconés et leurs mélanges.

Pour les tensio-actifs polymériques dérivés de polyoléfines, la partie apolaire est choisie parmi les polyoléfines telles que les polymères et/ou copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptedécène et de 1-octadécéne. Les chaînes de polymères sont hydrogénées ou non. Elles sont constituées d'au moins 40 carbones, et de préférence de 60 à 700 carbones.

La partie polaire de ces tensioactifs polymériques peut être anionique, cationique, non ionique, zwitterionique ou amphotère. Elle est par exemple constituée de dérivés acryliques, de polyalkylènes glycols ou de polyalkylène imine. Les tensioactifs polymériques à partie polaire acide carboxylique sont par exemple issus de la réaction d'une polyoléfine avec les acides carboxyliques tels que l'acide maléique, l'anhydride maléique, l'acide fumarique, l'acide itaconique, l'acide citraconique, l'acide mesaconique, l'acide aconitique.... De préférence, leur partie polaire est constituée par l'acide ou l'anhydride succinique, leurs dérivés esters ou amides, les sels d'ions alcalins, alcalino-terreux, ou organiques correspondants, ou bien encore par du polyoxyéthylène.

Les tensioactifs polymériques dérivés de polyoléfines sont également choisis parmi les dérivés polyoléfines d'acide succinique décrits dans les brevets US4234435, US4708753, US5129972, US4931110, GB2156799 et US4919179. La partie polyoléfine peut être constituée de polyisobutylène, hydrogéné ou non. L'anhydride ou l'acide succinique peuvent être modifiés par des alcools, des amines, des alcanols amines ou des polyols, ou encore se trouver sous forme de sels d'ions alcalins ou alcalino-terreux, ou encore d'ions organiques comme les ions diéthanolammonium ou triéthanolammonium. On peut citer notamment les polyisobutylène à terminaison succinique modifiée, tels que les produits commercialisés sous les dénominations L2724, L2721, L2722, OS156565 et Lubrizol 5603 par la société Lubrizol ou encore le Chemcinnate 2000 fourni par la société Chemron. Un autre exemple de tensioactif polymérique utilisable dans l'invention est le produit de la réaction de l'anhydride maléique avec du polyisobutylène, tel que le Glissopal SA commercialisé par BASF.

Les émulsionnants siliconés peuvent être choisis par exemple dans le groupe comprenant les diméthicone copolyols, les alkyldiméthicone copolyols pouvant comporter des hétéroatomes tels que le fluor, et les mélanges les contenant, par exemple le mélange de Polyglyceryl-4 isostearate/Cetyl dimethicone copolyol/Hexyl laurate vendu sous la dénomination Abil WE 09 par la société Goldschmidt, le Cetyl Dimethicone Copolyol vendu sous la dénomination Abil EM 90 par la société Goldschmidt et le mélange de Cyclomethicone/Dimethicone Copolyol vendu sous la dénomination Q2-3225C par la société Dow Corning. Le taux d'émulsionnant dans l'émulsion primaire va en général de 0,1 à 10 % en poids de matière active et de préférence de 0,5 à 5 % en poids par rapport au poids total de l'émulsion primaire.

Les compositions de l'invention peuvent contenir tous les additifs habituellement utilisés en cosmétique et trouveront des applications dans le domaine du Soin, du Maquillage et des produits Solaires.

Les compositions aqueuses conformes à la présente invention peuvent comprendre en outre des adjuvants cosmétiques classiques notamment choisis parmi les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les Carbopols (Carbomers) et les Pemulen (Copolymère acrylate/C10-C30-alkylacrylate) ; le terpolymère acide méthacrylique/acrylate de methyle/diméthyl meta-isoprpenyl benzyl isocyanate d'alcool ethoxylé (Nom INCI : Polyacrylate-3) comme le produit vendu par Amerchol sous le nom VISCOPHOBE DB 1000 ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les polymères synthétiques tels que le poly C10-30 alkyl acrylate vendu sous la dénomination « Doresco IPA 13-1 » par la société Landec ou encore les argiles modifiées telles que le l'hectorite et ses dérivés, comme les produits commercialisés sous les noms de Bentone.

Les solvants organiques peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de myrtrimonium), le bromure de dodécyl-triméthylammonium, le bromure d'hexadécyltriméthylammonium, et leurs mélanges tel que le mélange vendu sous la dénomination Cetrimide® par la société FEF CHEMICALS.Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Comme charges qui peuvent être utilisées dans la composition de l'invention, on peut citer par exemple, les pigments ; la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microsphères à base de copolymères acryliques, telles que celles en copolymère diméthacrylate d'éthylène glycol/methacrylate de lauryle vendues par la société Dow Corning sous la dénomination de POLYTRAP ; les poudres de polymethylmethacrylate telles que celles coomercialisées sous la dénomination MICROPEARL M 100 par la société Matsumoto ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les poudres de polyuréthanne telles que la poudre de copolymère hexaméthylène diisocyanate/trimethylol hexyllactone commercialisée sous la dénomination Plastic Powder D-400 par la société Toshiba Pigment (Nom CTFA : HDI / Trimethylol Hexyllactone Crosspolymer) ; et leurs mélanges. Quand elles sont présentes, ces charges peuvent être en des quantités allant de 0,001 à 20 % en poids, de préférence de 0,1 à 10 % en poids et mieux de 1 à 5 % en poids par rapport au poids total de la composition.

Parmi les actifs, on peut citer :
- les vitamines (A, C, E, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

La composition selon l'invention peut constituer un produit pour le soin de la peau, notamment pour le visage, le cou, le contour de l'oeil, le corps ; ou bien encore un produit de maquillage tel qu'un produit du teint (notamment fond de teint), produit anti-cernes, , un produit de protection solaire ou bien un produit de nettoyage de la peau. De manière préférentielle La composition selon l'invention sera un produit de protection solaire.

Les compositions cosmétiques selon l'invention peuvent par exemple être utilisées comme produit de soin et/ou de protection solaire pour le visage et/ou le corps de consistance liquide à semi-liquide, telles que des lotions, des laits, des crèmes plus ou moins onctueuses, des gels, des gel-crèmes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations solaires suivantes ; les quantités sont indiquées en pourcentages en poids :

### Exemples :

| **Phase** | **Constituants** | **Formule (hors invention)** | **Exemple 1** | **Exemple 2** |
|---|---|---|---|---|
| A | Eau distillée | 15.375% | 15.375% | 15.375% |
| | Conservateurs | 0.8% | 0.8% | 0.8% |
| | Glycérine | 5.00% | 5.00% | 5.00% |
| | EDTA | 0.20% | 0.20% | 0.20% |
| B | Distéarate de sucrose | 2.00% | 2.00% | 2.00% |
| | Stéarate de sorbitan 20 OE | 1.00% | 1.00% | 1.00% |
| | N Stéaroyl L glutamique acide mono sodique | 0.75% | 0.75% | 0.75% |
| | Conservateur | 0.15% | 0.15% | 0.15% |
| | Cyclohexasiloxane | 1.72% | 1.72% | 1.72% |
| | Diisopropyl sebacate | 2.71 % | 2.71 % | 2.71 % |
| | Isononyl isononanoate | 5.57% | 5.57% | 5.57% |
| | Octocrylène | 7.00% | 7.00% | 7.00% |
| | Butyl methoxydibenzoyl méthane | 3.00% | 3.00% | 3.00% |
| | Ethyl hexyl salicylate | 5.00% | 5.00% | 5.00% |
| C | Eau distillée | 15.00% | 15.00% | 15.00% |
| | Carbomer 980 | 0.30% | 0.30% | 0.30% |
| | Triéthanolamine | 0.30% | 0.30% | 0.30% |
| D | Eau distillée | 6.00% | 6.00% | 6.00% |
| | Gomme de Xanthane | 1.60% | 1.60% | 1.60% |
| E | PEG 12 dimethicone | 0.50% | 0.50% | 0.50% |
| F | Aluminium starch octenylsuccinate | 3.00% | 3.00% | 3.00% |
| G | Takemoto NLK 506 | 0 | 3.00% | 5.00% |
| H | Eau distillée | QSP 100 | QSP 100 | QSP 100 |
| **SPF in vitro** | | **14.4 +/- 0.9** | **24.3 +/- 3.1** | **33.8 +/-3.7** |

Le facteur de protection solaire (SPF) est déterminé selon la méthode « in vitro » décrite par B.L. Diffey dans J. Soc. Cosmet. Chem. 40 , 127-133, (1989). Les mesures ont été réalisées à l'aide d'un spectrophotomètre UV-1000S de la société Labsphere. Chaque composition est appliquée sur une lame de quartz, sous la forme d'un dépôt homogène et régulier à raison de 1.33 mg/cm2.

On constate que le SPF est significativement amélioré lorsque des particules hémisphériques creuses sont ajoutées dans la composition de référence. Respectivement, à 3 et 5% de particules Takemoto NLK 506, les SPF vitro est amélioré de 68% et de 134%.Par ailleurs, ces particules permettent de réduire significativement le brillance, après application, des formules filtrantes les contenant.

## Revendications

1. Utilisation de particules concaves ou annulaires de matériau siliconé, notamment sous forme de portions de sphères creuses dans une composition comprenant dans un milieu physiologiquement acceptable au moins une phase aqueuse et au moins un filtre UV organique et/ou un filtre UV minéral, dans le but d'augmenter le facteur de protection solaire (FPS).

2. Utilisation selon la revendication 1, où les particules de matériau siliconé sont en association avec au moins un agent mouillant.

3. Utilisation selon la revendication 1 ou 2, **caractérisée en ce que** les particules concaves de matériau siliconé ont un diamètre moyen inférieur ou égal à 10 µm.

4. Utilisation selon la revendication 3, **caractérisée en ce que** les particules concaves de matériau siliconé ont un diamètre moyen allant de 0,1 µm à 8 µm,

5. Utilisation selon l'une quelconque des revendications 1 à 4, **caractérisée en ce que** les particules concaves de matériau siliconé sont sous forme de portions de sphères creuses ayant une section transversale en forme de fer à cheval ou d'arceau.

6. Utilisation selon l'une quelconque des revendications précédentes, **caractérisée en ce que** le matériau siliconé est un polysiloxane réticulé de structure tridimensionnelle comprenant, ou constitué de, des motifs de formule (I) : SiO₂ et de formule (II) : R¹SiO_{1,5}
dans lesquelles R¹ désigne un groupe organique ayant un atome de carbone directement relié à l'atome de silicium.

7. Utilisation selon la revendication 6, **caractérisée en ce que** R¹ est un groupe alkyle en C₁-C₄ ou un groupe phényle.

8. Utilisation selon la revendication 7, **caractérisée en ce que** R¹ est un groupe méthyle.

9. Utilisation selon la revendication 6, **caractérisée en ce que** R¹ est choisi parmi les groupe époxy, (méth)acryloxy, alkényle, mercaptoalkyle, aminoalkyle, halogénoalkyle, glycéroxy, uréido, cyano.

10. Utilisation selon l'une quelconque des revendications 5 à 9, **caractérisée en ce que** le matériau siliconé comprend les motifs (I) et (II) selon un rapport molaire motif (I) / motif (II) allant de 30/70 à 50/50, de préférence allant de 35/65 à 45/55.

11. Utilisation selon l'une quelconque des revendications précédentes, **caractérisée en ce que** les particules concaves en matériau siliconé sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

12. Utilisation selon la revendication 11, **caractérisée en ce que** dans l'étape (a), le rapport molaire du composé (III) au composé (IV) va de 30/70 à 50/50.

13. Utilisation selon la revendication 11 ou 12, **caractérisée en ce que** le rapport en poids de l'eau sur le total des composés (III) et (IV) va de 10/90 à 70/30 dans l'étape (a).

14. Utilisation selon la revendication 11, **caractérisée en ce que** R est un groupe alkyle en C₁-C₄, ou un groupe phényle.

15. Utilisation selon la revendication 14, **caractérisée en ce que** R est un groupe méthyle.

16. Utilisation selon la revendication 11, **caractérisée en ce que** R est choisi parmi les groupes époxy, (méth)acryloxy, alkényle, mercaptoalkyle, aminoalkyle, halogénoalkyle, glycéroxy, uréido, cyano.

17. Utilisation selon l'une quelconque des revendications précédentes, **caractérisée en ce que** les particules concaves sont formées d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

18. Utilisation selon la revendication 1 ou 2, **caractérisée en ce que** les particules de forme annulaire présentent un diamètre moyen extérieur de 0,05 à 15 µm et un diamètre moyen intérieur de 001 à 10 µm; la différence entre le diamètre moyen extérieur et le diamètre moyen intérieur étant de 0,04 à 5µm.

19. Utilisation selon la revendication 18, présentent un réseau polysiloxane comprenant des unités siloxane de formule (1), (2) (3) (4) (5) et (6)
SiO_{4/2} (1)
Si(OH)_{3/2} (2)
R₁(SiO_{3/2} (3)
R₂SiO_{3/2} (4)
R₃SiO_{3/2} (5)
R₄SiO_{3/2} (6)
dans lesquelles R₁ et R₃ désignent des groupes alkyle, cycloalkyle, aryle, alkylaryle ou aralkyle, et R₂ et R₄ désignent chacun un groupe hydrocarboné choisi parmi les groupes acryloxy, methacryloxy, vinyle ou mercapto ;
le rapport molaire unités siloxane de formule (1) / unités siloxane de formule (2), (3), (4), (5) et (6) étant de 20/80 à 50/50 ;
le rapport molaire unités siloxane de formule (2), (3) et (4) / unités siloxane de formule 5) et (6) étant de 50/50 à 75/25;
le rapport molaire unités siloxane de formule (3) et (5) / unités siloxane de formule (4) et (6) étant de 20/80 à 60/40.

20. Utilisation selon l'une quelconque des revendications précédentes, où l'agent mouillant est choisi parmi les silicones hydrosolubles comportant au moins un groupement polyoxyalkyléné monovalent terminal ou pendant.

21. Utilisation selon la revendication 20, où l'agent mouillant est choisi parmi les silicones hydrosolubles comportant au moins un groupement polyoxyalkyléné de formule générale (a) suivante
R²₃SiO(R²₂SiO)ₚ(R²PESiO)_{q}SIR²₃ (a)
dans laquelle
- les radicaux R², identiques ou différents, désignent un radical hydrocarboné monovalent choisis parmi les radicaux alkyles, aryles et aralkyles ayant au plus 10 atomes de carbone ; certains des radicaux R² peuvent également contenir en plus un groupe ethylcyclohexylenemonoxyde de formule 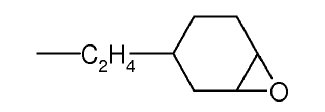 et sont en faible proportion dans la chaîne polysiloxane ;
- p varie de 0 à 150, de préférence de 0 à 100 et plus préférentiellement de 0 à 30
- q varie de 1 à 12, de préférence de 1 à 10 et plus préférentiellement de 1 à 8.
- le groupe polyéther PE a la formule (b) suivante
-CₓH₂ₓ(OC₂H₄)_{y}(OC₃H₆)_{z}OR³ (b)
dans laquelle :
x varie de 1 à 8 et de préférence varie de 2 à 4 et plus préférentiellement est égal à3;
y est supérieur à 0
z est supérieur ou égal à 0 ; les valeurs de y et z sont tels que le poids moléculaire total de la portion polyoxyalkylénée du groupe polyéther PE varie de 200 à 10.000 et plus préférentiellement de 350 à 4000 ;
R³ désigne hydrogène, un groupe alkyle en C₁-C₈ ou un groupe acyle en C₂-C₈ .

22. Utilisation selon la revendication 21, où les radicaux R² sont choisis parmi les alkyles inférieurs en C₁-C₆ ; phényle et benzyle.

23. Utilisation selon la revendication 22, où les radicaux R² sont choisis parmi les alkyles inférieurs en C₁-C₄ et encore plus particulièrement désignent méthyle.

24. Utilisation selon l'une quelconque des revendication 20 à 23, où les radicaux R³ sont choisis parmi les alkyles inférieurs en C₁-C₄ et encore plus particulièrement désignent méthyle.

25. Utilisation selon l'une quelconque des revendications 20 à 24, où les silicones hydrosolubles de formule (a) sont choisies parmi celles de formule (a') suivante:
MeSiO(MeSiO)ₚ(MePESiO)_{q}SlMe₃ (a')
où Me désigne méthyle; PE désigne le groupe de formule (b') :
-(CH₂)₃O(OC₂H₄)_{y}(OC₃H₆)_{z}OR³ (b')
où x, z et z ont les mêmes valeurs indiquées dans la revendications 29 et R³ désigne hydrogène ou un groupe alkyle en C₁-C₄. et plus particulièrement methyle

26. Utilisation selon la revendication 20, où la silicone hydrosoluble est choisie parmi les silicones ramifiées de formule (c) suivante :
(MeSiO)_{q-2}[SiOMe2)_{p/q} OPE]q (c)
où
- p varie de 0 à 150, de préférence de 0 à 100 et plus préférentiellement de 0 à 30
- q varie de 1 à 12, de préférence de 1 à 10 et plus préférentiellement de 1 à 8.
- Me signifie méthyle ;
- PE désigne le groupe de formule (d) suivante :
-(OC₂H₄)_{y}(OC₃H₆)_{z}R³ (d)
où
- y est supérieur à 0
- z est supérieur ou égal à 0 ; les valeurs de y et z sont tels que le poids moléculaire total de la portion polyoxyalkylénée du groupe polyéther PE varie de 200 à 10.000 et plus préférentiellement de 350 à 4000 ;
- R³ désigne un groupe alkyle en C₁-C₄.

27. Utilisation selon l'une quelconque des revendications précédentes , où le filtre UV organique est choisi parmi les anthranilates ; les dérivés de dibenzoylméthane ; les dérivés cinnamiques ; les dérivés salicyliques, les dérivés du camphre ; les dérivés de la benzophénone ; les dérivés de β , β - diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

28. Composition selon la revendication 27, où le filtre UV organique est choisi parmi
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine
la 2,4,6-tris(biphenyl)-1,3,5-triazine)
la 2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

29. Utilisation selon l'une quelconque des revendications précédentes, où les filtres UV inorganiques sont choisis parmi les pigments d'oxyde métallique, enrobés ou non enrobés.

30. Utilisation selon la revendication 29, où lesdits pigments sont choisis parmi les oxydes de titane, de zinc, de fer, de zirconium, de cérium et leurs mélanges, enrobés ou non.

31. Utilisation selon la revendication 30, **caractérisée en ce que** les pigments sont à base d'oxyde de titane, enrobé ou non enrobé.

32. Utilisation selon la revendication 31, **caractérisée en ce que** l'oxyde de titane est sous forme rutile, anatase ou amorphe.

33. Utilisation selon l'une quelconque des revendications 29 à 32, **caractérisée en ce que** pigments ont une taille moyenne de particule élémentaire supérieure à 5 nm et inférieure à 100 nm.

34. Utilisation selon l'une quelconque des revendications 29 à 33, **caractérisée en ce que** la composition est sous forme d'émulsion huile-dans-eau, d'émulsion eau-dans-huile, d'émulsion triple eau-dans-huile-dans-eau ou huile-dans-eau-dans-huile.

35. Utilisation selon la revendication 34, **caractérisée en ce que** la composition est sous forme d'émulsion huile-dans-eau ou d'émulsion triple eau-dans-huile-dans-eau.

36. Composition comprenant dans un milieu physiologiquement acceptable :
a) au moins une phase aqueuse
b) des particules concaves ou annulaires de matériau siliconé, notamment sous forme de portions de sphères creuses telles que définies dans les revendications précédentes ;
c) au moins un filtre UV organique et/ou un filtre UV minéral tel que défini dans les revendications précédentes;
d) au moins un agent mouillant tel que défini dans les revendications précédentes.

37. Composition selon la revendication 36, **caractérisée en ce qu'**elle est sous forme d'émulsion huile-dans-eau, d'émulsion eau-dans-huile, d'émulsion triple eau-dans-huile-dans-eau ou huile-dans-eau-dans-huile.

38. Composition selon la revendication 36 ou 37, **caractérisée en ce qu'**elle est sous forme d'émulsion huile-dans-eau ou d'émulsion triple eau-dans-huile-dans-eau.

39. Composition selon l'une quelconque des revendications 36 à 38, **caractérisée en ce que** les particules concaves ou annulaires sont présentes en une teneur allant de 0,1 à 15 % en poids par rapport au poids total de la composition.

40. Composition selon l'une quelconque des revendications 36 à 39, où le ou les filtres UV sont présents dans des proportions allant de 0,01 à 20% en poids par rapport au poids total de la composition, et de préférence allant de 0,1 à 10% en poids par rapport au poids total de la composition.

41. Composition selon l'une quelconque des revendications 36 à 40, où la proportion massique de la phase aqueuse varie de 25 à 95%, de préférence de 30 à 90% et de façon encore plus préférentielle de 40 à 80% en poids par rapport au poids total de la composition.

42. Composition selon l'une quelconque des revendications 36 à 41, où l'agent mouillant est choisi parmi les silicones hydrosolubles comportant au moins un groupement polyoxyalkyléné monovalent terminal ou pendant.

43. Composition selon l'une quelconque des revendications 36 à 42, comprenant en plus au moins un adjuvant cosmétique choisi parmi les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

44. Composition selon l'une quelconque des revendications 36 à 43, **caractérisée par le fait qu'**elle se présente sous forme de crème blanche ou colorée, de lait, de lotion, de gel-crème, sous forme solide.

45. Composition selon l'une quelconque des revendications 36 à 44, **caractérisée par le fait qu'**elle est conditionnée dans un aérosol et peut former une mousse ou un spray.

46. Utilisation d'une composition telle que définie dans l'une quelconque des revendications 36 à 45 pour la fabrication de produits pour le traitement cosmétique de la peau, des lèvres, des ongles, des cheveux, des cils, sourcils et/ou du cuir chevelu.

47. Utilisation d'une composition telle que définie dans l'une quelconque des revendications 36 à 45 pour la fabrication d'un produit de soin et/ou de protection solaire pour le visage et/ou le corps.

48. Utilisation d'une composition telle que définie dans l'une quelconque des revendications 36 à 45 pour la fabrication d'un produit de maquillage.
